Europäisches Patentamt

European Patent Office

Office européen des brevets

(11) Publication number: **0 250 034**
**A1**

# EUROPEAN PATENT APPLICATION

(21) Application number: **87201105.1**

(22) Date of filing: **11.06.87**

(51) Int. Cl.4: **A61B 5/02** , **A61M 25/00**

(30) Priority: **12.06.86 NL 8601527**

(43) Date of publication of application:
**23.12.87 Bulletin 87/52**

(84) Designated Contracting States:
**DE FR GB IT NL**

(71) Applicant: **Sentron v.o.f.**
**Oosteinde 8**
**NL-9301 LJ Roden(NL)**

(72) Inventor: **Schepel, Siebe Johannes**
**Swarte Ruiters 29**
**NL-9351 NL Leek(NL)**
Inventor: **Ligtenberg, Hendrikus Cornelis**
**Johannes Vermeerstraat 15**
**NL-9312 PZ Nietap(NL)**

(74) Representative: **Smulders, Theodorus A.H.J.**
**et al**
**Vereenigde Octrooibureaux Nieuwe Parklaan**
**107**
**NL-2587 BP 's-Gravenhage(NL)**

(54) Catheter system for sensor assemblies.

(57) A catheter system and method are provided for
for example determining dynamic blood pressure
measurements and for simultaneously conducting
one other in vivo medical procedure. The catheter
system includes a cannula (12) and a catheter (13)
having a distal tip portion sensor (14) as well as a
tail portion (15) that is of reduced diameter and that
defines, with the cannula, an elongated annular pas-
sageway (21) for the transmission of dynamic blood
pressure, for the taking of blood samples, for the
administration of medicaments to the bloodstream,
and the like. The catheter system is thin enough to
be utilized with the arteria radialis.

FIG 1

# CATHETER SYSTEM FOR SENSOR ASSEMBLIES

## Background and Description of the Invention

The invention generally relates to a catheter system, more particularly to a catheter system including a catheter tube to be inserted into a blood vessel, an inserting cannula, and a sensor assembly mounted in the tip of the catheter tube, which sensor assembly includes one or more sensor components and one or more conduction means through which the sensor assembly is actuated and the signal produced by the sensor assembly is applied to a signal processing unit.

Catheterization is a known method for effecting in vivo medical examination procedures such as those conducted for monitoring purposes. In such a method, a sufficiently flexible, tubular catheter having a sensor assembly at its tip is inserted into a bloodstream. An example of a suitable sensor assembly is one including a sensor component that is an Ion Sensitive Field Effect Transistor (known as an ISFET) for determining an important chemical parameter, such as the activity of an ion, for example an $H^+$ ion, to which the ISFET is selectively sensitive. When the sensor component interacts with the blood within the blood vessel, the sensor assembly produces an electrical signal which can be transmitted or applied for further processing to a measuring system exterior of the patient through current conducting wires that are connected to the sensor components and that extend through the catheter.

To minimize the trauma experienced by the patient on account of the use of a catheter system and its insertion into the bloodstream, there is a need to provide catheter systems that are as thin as possible. It is generally preferred that such catheters be suitable for insertion into a narrow peripheral artery, such as the so-called arteria radialis. Usually a percutaneous technique is used for inserting the catheter into an artery, in which case the catheter extends through an inserting cannula via a hemostat valve into the bloodstream.

In order to provide a catheter system that is as diverse as possible in its usefulness and that fulfills the need for a system that is capable of performing a number of different measurements by means of one and the same catheter system, the present invention exhibits a basic design that can be used for a plurality of purposes. For example, in addition to determining the activity of an ion, such as that of the $H^+$ ion or pH, the invention has means, for example, for performing a blood pressure measurement for determining the dynamic blood pressure.

By known devices, such a blood pressure measurement typically is performed by feeding an external pressure sensor through a liquid-filled channel, either through a lumen in the catheter or through the space between the catheter and the cannula, in order to bring the blood pressure sensor into contact with the blood. When using such catheter systems in relatively small blood vessels, however, there is the problem of a possible conflict between the desire to concurrently measure the blood pressure and the desire for an optically small dimension of catheter/cannula system. This is primarily because the catheter has to house a sensor too.

In these instances, there is the desire to minimize the cannula diameter so as to reduce trauma. There is also the concurrent desire to maximize the space in hydraulic channels in order to better accommodate means for pressure measurement or the like. Because of these competing objectives, the possibility of having the catheter also house a sensor becomes exceedingly problematical, inasmuch as these objectives would seem to require too drastic of a miniaturization of the sensor assembly or components.

## Summary of the Invention

These advantages, needs and objectives are achieved by providing a catheter system for sensor assemblies that has a minimal diametric size with a maximal hydraulic channel space. The catheter system according to the present invention is provided with a catheter having a sensor component mounted at its distal tip, as well as means for determining dynamic blood pressure or the like, which means includes a member hydraulically conducting the dynamic blood pressure and having contact means to be brought into contact with the bloodstream. The hydraulically conducting member includes a cylindrical passageway formed between the internal surface of cannula and a diameter reduced over a portion of its length, which reduced diameter portion extends upstream and downstream of the contact means located generally at the distal end portion of the cannula. The invention also includes an improved and efficient method for medical in vivo use of the catheter system for monitoring of body parameters through the simultaneous use of a distal tip sensor component and a cylindrical channel that is generally coaxial with the sensor component and that is used to achieve fluid flow functions such as the measurement of dynamic blood pressure, the taking of blood samples, the administration of medicaments to the bloodstream, and the like.


Brief Description of the Drawings

The invention will now be described in greater detail hereinafter with reference to an embodiment of the catheter system shown in the drawings, in which:
Figure I schematically shows in cross-sectional view a catheter tube inserted in vivo through an inserting cannula into the arteria radialis; and

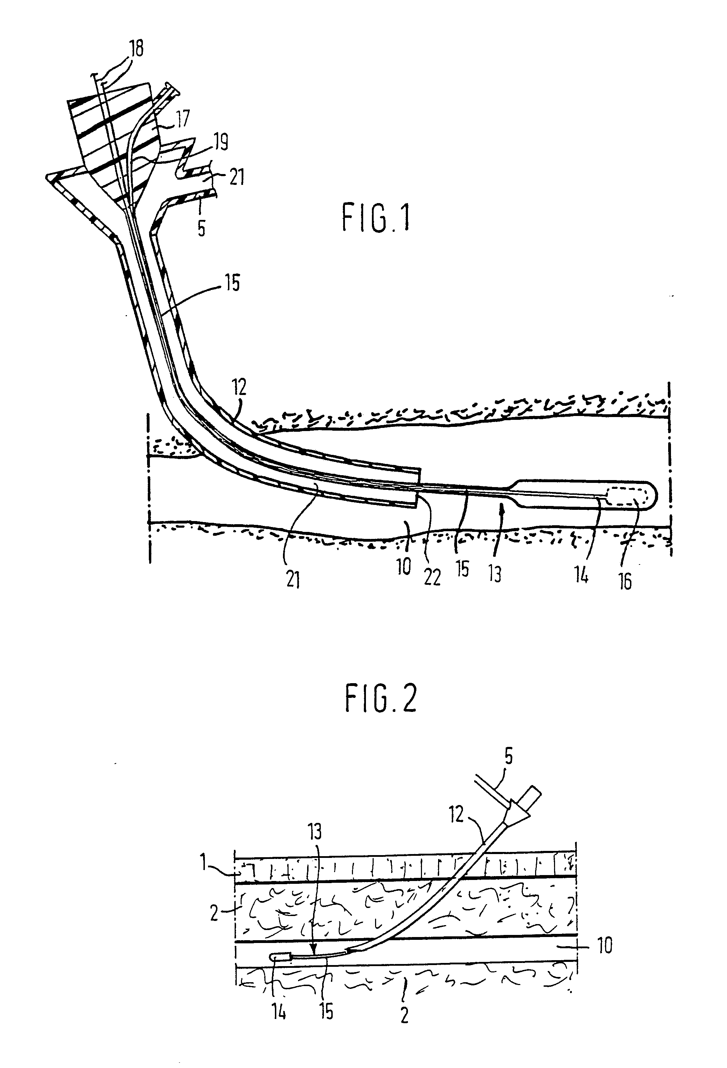

Figure 2 shows the cannula of Figure I in elevational side view and provided with a stub for connection of a pressure conducting member thereto.


Description of the Preferred Embodiments

Reference numeral I0 in Figure I denotes the arteria radialis in the region of the wrist of an arm of a patient, while I2 denotes the inserting cannula inserted from outside into the arm of the patient to extend in artery I0. A hemostat valve of known construction (not shown) prevents blood from being forced by the blood pressure to squirt from the cannula I2.

A catheter assembly I3 includes a tip portion I4 of sufficient diameter and length to accommodate one or more sensor components I6 that are housed therein in known, conventional manner. Catheter tip I4 is an extension of and merges into the distal portion of tail I5 of the catheter, which tail is of considerably smaller cross-section than the rest of the catheter I7 and has a diameter determined only by the requirement of being sufficient to accommodate current conducting wires I8 or one or more optical fibres leading to and from the sensor component I6 and, when needed for a particular sensor component, a canal I9.

The arteria radialis I0 shown in Figure 1 has a diameter of about 2 mm. Taking this dimension as the starting point, the enlarged catheter tip I4 has a diameter of about 0.85 mm, the tail I5 of the catheter has a diameter of about 0.55 mm, and the cannula I2 has an internal diameter of 0.9 mm (18 G).

In Figure 2, components corresponding to those shown in Figure I are identified by like reference numerals. Furthermore, I denotes human skin and 2 denotes tissues thereunder. A stub 5 is attached to the cannula I2 and is connected in a manner not shown to a tube leading to a suitable measuring device (not shown), which can contain a physiological saline solution.

When this suitable measuring device is a blood pressure monitoring system, the dynamic blood pressure propagating member has contact means to be brought into contact with the bloodstream in the form of a channel 2I filled with, for example, a physiological salt solution, which channel 2I opens freely into the bloodstream I0 but is connected to a part of the inserting cannula I2 exterior of the patient, for example to the stub 5 on the sidewall of the cannula I2. In this embodiment, the portion of reduced external diameter of the catheter tube should extend at least for the entire length of the cannula I2 as generally illustrated in Figure I.

By reducing the external diameter of the catheter tube I8 to form the tail I5 that extends to its free or termination end at which the pressure conducting member is positioned in the bloodstream, sufficient space is provided between the external wall of the catheter at its tail I5 and the inner wall of the cannula I2 to ensure an accurate measurement with respect to fluid flowing therethrough, such as blood during the course of blood pressure measurement.

With further reference to the catheter tube suitable for use in the catheter system according to the invention, same is structured such that its reduced diameter tail I5 extends upstream and downstream of the contact means or port 22 of the cannula I2. This structure ensures that the enlarged catheter tip I4 will not interfere with the flow of fluid through the contact means or port 22. When the sensor component or components I6 are in the nature of an ISFET for measuring ion concentration such as pH, pK, pNa and the like, then the tail I5 need be no larger than that required to accommodate wires or conductors I8. When the sensor component or components I6 are of the type that include pressure transducer components, then the tail I5 will also accommodate canal I9 to apply an atmospheric air reference pressure to the sensor.

Further regarding the catheter assembly I3, a pressure transducer sensor component outside the patient's body may be used for measuring the dynamical blood pressure via the channel 2I as indicated earlier. The arrangement shown in the drawings allows for the enlarged annular space between the cannula I2 and the catheter tail I5 to be generally used as a pathway for various purposes of communication between the bloodstream and devices outside of the body. Such pathway could be used, for example, for taking a blood sample, for administering fluid drugs, and the like. The structure of the invention allows for multipurpose communication between the bloodstream and the outside world, which multipurpose functionality is independent of the type of sensor housed in the catheter tip 14.

While particular embodiments of the invention have been shown and described, it will be obvious to those skilled in the art that changes and modifications may be made therein without departing from the invention in its broader aspects, and, therefore, the aim in the appended claims is to cover all changes and modifications as fall within the true spirit and scope of the invention.

## Claims

1. A catheter system for simultaneously conducting at least two in vivo procedures, comprising a catheter tube to be inserted into a blood vessel, an inserting cannula and a sensor assembly mounted in the tip of the catheter tube, which sensor assembly includes a sensor component for determining a blood parameter as the first in vivo procedure and one or more conduction means through which the sensor component is actuated and the signal produced by the sensor component is applied to a signal processing unit, said catheter system besides said sensor component is further provided with means for carrying out the other in vivo procedure, said carrying out means having contact means for hydraulically making contact with the blood stream, the catheter tube having its external diameter reduced over a tail portion of its length, which tail portion extends upstream and downstream of the inserting cannula.

2. The catheter system according to claim 1, wherein said tip of the catheter tube has a diameter large enough to accomodate said sensor component.

3. The catheter system according to claim 1, wherein the catheter system being provided with means for determining dynamic blood pressure within the blood vessel, said determining means including a member for hydraulically conducting the dynamic blood pressure, said member having the contact means for making contact with the blood stream.

The catheter system according to claim 1, wherein the sensor component in the tip of the catheter tube is selected from the group consisting of a pressure transducer for the in vivo determination of the dynamic blood pressure and a sensor component for the in vivo determination of one or more chemical parameters of the blood such as pH, pK, PNa and the like.

5. The catheter system according to claim 1, wherein the sensor component in the tip of the catheter is for determining the one or more chemical parameters of the blood and the member for hydraulically conducting the dynamic blood pressure is an annular passageway between the internal surface of the cannula and the external surface of the reduced diameter tail portion to the catheter tube.

6. The catheter system according to claim 1, wherein said tail portion reduced external diameter is sized to accommodate only said conduction means.

7. The catheter system according to claim 1, wherein the sensor component in the tip of the catheter is a pressure transducer for determining the dynamic blood pressure and wherein said tail portion reduced external diameter is sized to accommodate only said conduction means and a canal to apply an atmospheric air reference pressure to said pressure transducer.

8. The catheter system according to claim 1, wherein said catheter system is sized and structured to be inserted into the arteria radialis .

9. An in vivo method for simultaneously conducting at least two in vivo procedures comprising

inserting a cannula into a blood vessel at a location at which the two in vivo procedures are desired;

inserting a catheter tube within and through said cannula, said catheter tube having a distal tip sensor component and an elongated tail portion having an external diameter that is reduced with respect to the distal tip sensor component, said catheter tube inserting step including providing an elongated annular passageway between the cannula and the elongated tail portion;

carrying out one of the in vivoprocedures via hydraulically making contact with the blood in the blood vessel along the catheter tube; and

simultaneously carrying out the another in vivo procedure via hydraulically making contact of the blood in the blood vessel with the sensor component in the distal end of the catheter tube.

10. The method according to claim 9, wherein one of the said in vivo procedures includes a dynamic blood pressure determining step by hydraulically communicating the blood in said blood vessel to a pressure transducer comprising device through said elongated annular passageway, and wherein said another in vivo procedure includes determining one or more chemical parameters by hydraulically contacting the blood with the distal tip sensor.

11. The method according to claim 9, wherein said catheter tube inserted within and through said cannula has a pressure transducer as a distal tip sensor component and one of the said in vivo procedures includes a dynamic blood pressure determining step by hydraulically communicating the blood in said blood vessel to the pressure transducer in the distal end of the catheter, and wherein said another in vivo procedure includes taking a blood sample through the annular space between the cannula and the catheter tail.

12. The method according to claim 11, wherein said another in vivo procedure includes administering a fluid drug through the annular space between the cannula and the catheter tail.

13. The method according to claim 8, wherein the blood vessel is the arteria radialis.

FIG.1

FIG.2

European Patent Office

**PARTIAL EUROPEAN SEARCH REPORT**
which under Rule 45 of the European Patent Convention shall be considered, for the purposes of subsequent proceedings, as the European search report

Application number

EP 87 20 1105

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 593 701 (KOBAYASHI)<br><br>* Column 6, lines 19-38; figures 6a,6b * <br><br>--- | 1-3 | A 61 B 5/02<br>A 61 M 25/00 |
| X | INTERNATIONAL ELECTRONICS, vol. 14, no. 2, April/May 1968, page 23 "Tiny heart probes may aid doctors"<br><br>* Whole article * <br><br>---------- | 1-3 | |

TECHNICAL FIELDS SEARCHED (Int. Cl.4)

A 61 B

A 61 M

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8

Claims searched incompletely:

Claims not searched: 9-13

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 23-09-1987 | VANRUNXT |

EPO Form 1505.1.03.82